(19) Europäisches Patentamt
European Patent Office
Office européen des brevets

(11) Veröffentlichungsnummer: **0 416 282 B1**

# (12) EUROPÄISCHE PATENTSCHRIFT

(45) Veröffentlichungstag der Patentschrift: **14.09.94**

(51) Int. Cl.[5]: **C12N 9/80**, C12P 13/24, //(C12N9/80,C12R1:05)

(21) Anmeldenummer: **90114653.0**

(22) Anmeldetag: **31.07.90**

## (54) Mikrobiologisch hergestellte N-Acyl-L-prolin-Acylase, Verfahren zu ihrer Gewinnung und ihre Verwendung.

(30) Priorität: **06.09.89 DE 3929570**

(43) Veröffentlichungstag der Anmeldung:
**13.03.91 Patentblatt 91/11**

(45) Bekanntmachung des Hinweises auf die Patenterteilung:
**14.09.94 Patentblatt 94/37**

(84) Benannte Vertragsstaaten:
**AT BE CH DE DK ES FR GB GR IT LI LU NL SE**

(56) Entgegenhaltungen:

**PATENT ABSTRACTS OF JAPAN, unexamined applications, C field, Band 4, Nr. 30,15. März 1980 THE PATENT OFFICE JAPANESE GOVERNMENT Seite 148 C 2**

**BIOCHIMICA ET BIOPHYSICA ACTA, Band 744, 1983, Amsterdam M. KIKUCHI et al."A new enzyme, proline acylase (N- Acyl-L-Proline Amidohydro- lase) frompseudomonas species" Seiten 180-188**

**PATENT ABSTRACTS OF JAPAN, unexamined applications, C field, Band 13, Nr. 278,26. Juni 1989 THE PATENT OFFICE JAPANESE GOVERNMENT Seite 68 C 611**

(73) Patentinhaber: **Degussa Aktiengesellschaft**
**Weissfrauenstrasse 9**
**D-60311 Frankfurt (DE)**

(72) Erfinder: **Groeger, Ulrich, Dr.**
**Löherstrasse 39 B**
**D-8750 Aschaffenburg (DE)**
Erfinder: **Leuchtenberger, Wolfgang, Dr.**
**Gronauer Strasse 10**
**D-4800 Bielefeld 1 (DE)**
Erfinder: **Drauz, Karlheinz, Dr.**
**Zur Marienruhe 15**
**D-6463 Freigericht 1 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein bisher nicht beschriebenes Enzym, das Umsetzungen folgender Art katalysiert:

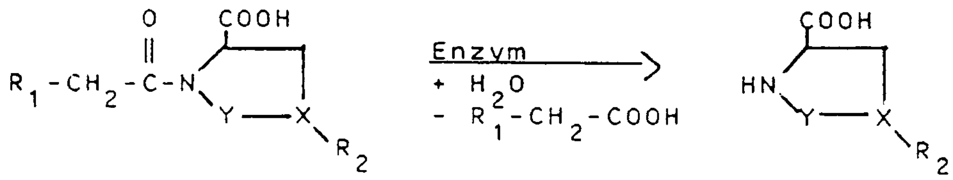


Das neue Enzym ist thermostabil und ergibt einen besonders guten Umsatz mit $R_1$ = H, X = CH und $R_2$ = H, mit $R_1$ = Cl, X = CH und $R_2$ = H, mit $R_1$ = H und X-$R_2$ = S, mit $R_1$ = Cl und X-$R_2$ = S, mit $R_1$ = H, X = CH, $R_2$ = H und Y = S und mit $R_1$ = Cl, X = CH, $R_2$ = H und Y = S.

Aus Biochimica et Biophysica Acta, 744 (1983), 180-188, Elsevier Biomedical Press, ist bereits eine Prolin - Acylase (N-Acyl-L-prolin-amido-hydrolase) aus einem Pseudomonas-Stamm bekannt. Das dort beschriebene Enzym wird jedoch bereits bei einer Temperatur von 50° C sehr rasch inaktiviert. Für eine industrielle Anwendung werden jedoch Enzyme benötigt, die eine hohe Stabilität aufweisen. Für die Reaktionsführung ist es insbesondere vorteilhaft, bei erhöhten Temperaturen zu arbeiten, welche die Reaktionsgeschwindigkeit und die Löslichkeit der Substrate wesentlich erhöhen. Das erfindungsgemäße Enzym ist wesentlich thermostabiler.

Es ist durch die folgenden Eigenschaften gekennzeichnet:

1) Reaktivität:
sie spaltet die Acetylgruppe von N-Acetyl-L-prolin ab, wobei Essigsäure und L-Prolin als Endprodukte entstehen, und kondensiert Essigsäure und L-Prolin, wobei N-Acetyl-L-prolin und Wasser als Endprodukte entstehen;
2) Substratspezifität:
sie hydrolisiert N-Acetyl-L-prolin,
N-Chloracetyl-L-prolin, N-Formyl-L-prolin,
N-Propionyl-L-prolin, N-Butyryl-L-prolin,
N-Valeryl-L-prolin, N-Caproyl-L-prolin,
N-Acetyl-L-4-hydroxyprolin,
N-Chloracetyl-L-thiazolidin-4-carbonsäure,
N-Chloracetyl-L-thiazolidin-2-carbonsäure,
N-Chloracetyl-L-pipecolinsäure,
N-Benzyloxycarbonyl-glycyl-L-prolin,
Glycyl-L-prolin, N-Acetyl-L-alanin,
N-Chloracetyl-L-methionin und
N-Chloracetyl-L-valin;
3) Optimaler pH-Wert:
der optimale pH-Wert ist 6,8 ± 0,5;
4) pH-Stabilität:
sie zeigt bei 22°C über einen Zeitraum von 3 Wochen eine gute Stabilität im pH-Bereich zwischen pH 7,0 und pH 10,0;
5) Optimale Temperatur:
die optimale Temperatur beträgt 65° C bei einem pH-Wert von 7,5;
6) Temperaturbeständigkeit:
bei 70° C und pH 7,5 ist nach 30-minütiger Inkubation kein Aktivitätsverlust nachweisbar;
7) Einflüsse von Inhibitoren und Aktivatoren:
inhibierend wirken vor allem 1,10-Phenanthrolin, 2-Mercaptoethanol, 4-Chloromercuribenzoat, 4-Hydroxymercuribenzoat, $Hg^{2+}$, $Cu^{2+}$, $Fe^{2+}$, $Fe^{3+}$, $Sn^{2+}$, $Zn^{2+}$ und $PO_4^{3-}$, aktivierend auf das Apoenzym wirken $Co^{2+}$ und $Zn^{2+}$;
8) Molekulargewicht:
das Molekulargewicht beträgt 380 000 ± 40 000 Dalton;
9) Untereinheiten:
das Molekül besteht aus 8 gleichgroßen Untereinheiten mit je 45 000 ± 5 000 Dalton;

10) $K_M$-Wert:

der $K_M$-Wert für das Substrat N-Acetyl-L-prolin beträgt 5 mM (30° C, 0,1 M Tris-HCl-Puffer, pH 7,0).

Die erfindungsgemäße N-Acyl-L-prolin-Acylase kann mittels des Comamonas testosteroni-Stammes DSM 5416 oder des Alcaligenes denitrificans-Stammes DSM 5417. gewonnen werden. Diese beiden Stämme wurden am 19.12.1988 bei der Deutschen Sammlung von Mikroorganismen und Zellkulturen GmbH (DSM) in Braunschweig hinterlegt.

Die folgenden Eigenschaften zeigen, daß der Stamm DSM 5416 der Species Comamonas testosteroni angehört:

Er wächst in 0,5 - 0,7 µm breiten und 1,5 - 3,0 µm langen, gramnegativen, durch mehr als eine polare Geißel beweglichen Stäbchen und bildet keine Sporen. Die Zellen werden durch 3 % KOH lysiert. Aminopeptidase (Cerny)-, Oxidase-, Katalase- und Phenylalanindeaminase-Reaktion sind positiv, Lecithinase- und Urease-Reaktion sind negativ. Das Wachstum ist strikt aerob, bei pH 5,6 auf Mac-Conkey-Agar und Simmons-Citrat-Agar positiv, bei 37° C und 41° C, SS-Agar und Cetrimid-Agar negativ. Pigmentbildung, ONPG-, ADH- und VP-Reaktion sind negativ. Nitratreduktion ist positiv, Denitrifikation ist negativ. Stärke, Gelatine, Casein, DNA, Tween 80 und Äsculin werden nicht hydrolisiert. Zum Wachstum werden Vitamine benötigt, der Tyrosin-Abbau ist positiv. Folgende Substrate werden zum Wachstum als Kohlenstoff- und Energiequelle verwertet: Gluconat, Glycerin, Pyruvat, L-Lactat, Malat, Adipat, Lävulinat, Mucat, D-Tartrat, Sebacinat, 2-Ketoglutarat, Acetat, Propionat, Butyrat, N-Acetyl-L-prolin, L-Prolin, L-Leucin, L-Aspartat, Norleucin und $\gamma$-Aminobutyrat. Kein Wachstum auf Arabinose, Glucose, Fructose, Lactose, Maltose, Mannose, Saccharose, Xylose, Mannit, 2-Ketogluconat, N-Acetylglucosamin, Caproat, Citrat, Glycolat, Malonat, Phenylacetat, L-Arginin, L-Histidin, L-Serin und L-Tryptophan.

Die folgenden Eigenschaften zeigen, daß der Stamm DSM 5417 der Species Alcaligenes denitrificans angehört:

Er wächst in 0,5 - 0,6 µm breiten und 1,0 - 2,0 µm langen, gramnegativen, durch peritrich angeordnete Geißeln beweglichen Stäbchen und bildet keine Sporen. Die Zellen werden durch 3 % KOH lysiert. Aminopeptidase (Cerny)-, Oxidase- und Katalase-Reaktion sind positiv, Phenylalanindeaminase-, Lecithinase- und Urease-Reaktion sind negativ. Das Wachstum ist aerob, bei pH 5,6, bei 37° C, auf Mac-Conkey-Agar, SS-Agar und Simmons-Citrat-Agar positiv, bei 41° C und auf Centrimid-Agar negativ. Pigmentbildung, ONPG-, ADH- und VP-Reaktion sind negativ. Nitratreduktion und Denitrifikation sind positiv. Als Speicherstoff wird Poly-$\beta$-hydroxybuttersäure (PHB) gebildet. Stärke, Gelatine, Casein, DNA, Tween 80 und Äsculin werden nicht hydrolysiert. Der Tyrosin-Abbau ist positiv. Folgende Substrate weden zum Wachstum als Kohlenstoff-und Energiequelle verwertet: Acetat, Adipat, Caproat, Citrat, L-Lactat, Malat, Propionat, Phenylacetat, Azelat, Gluconat, N-Acetylglucosamin, N-Acetyl-L-prolin, L-Prolin, L-Aspartat und L-Glutamat. Kein Wachstum auf Arabinose, Glucose, Fructose, Mannose, Maltose, Xylose, Mannit, 2-Ketogluconat, Glycolat, Lävulinat, Malonat, Oxalat, Meso-tartrat, Itaconat, Pimelat, Sebacinat, Suberat, L-Alanin und L-Serin.

Die Mikroorganismen können als lyophilisierte Kultur, durch Einfrieren bei -80° C oder in flüssigem Stickstoff bei -196° C aufbewahrt werden.

Arbeitskulturen werden auf Schrägagar-Röhrchen (Caseinpepton-Sojamehlpepton-Agar der Firma Merck AG, Darmstadt, FRG) gehalten.

Zur Gewinnung der erfindungsgemäßen N-Acyl-L-prolin-Acylase wird Comamonas testosteroni DSM 5416 oder Alcaligenes denitrificans DSM 5417 in einem wäßrigen Nährmedium, das eine Quelle für Kohlenstoff, Stickstoff, Mineralsalze und N-Acetyl-L-prolin als Induktor, bei Einsatz von Comamonas testosteroni DSM 5416 zusätzlich eine Vitaminquelle z.B. in komplexer Form als Hefeextrakt, enthält, bei einem Ausgang-pH-Wert zwischen 6,0 und 8,0 und einer Temperatur zwischen 25° C und 35° C aerob kultiviert, die Zellmasse abgetrennt und das Enzym aus den Zellen isoliert.

In größeren Mengen kann das Enzym beispielsweise so gewonnen werden, daß man Comamonas testosteroni DSM 5416 oder Alcaligenes denitrificans DSM 5417 in an sich bekannter Weise in einem Bioreaktor gewünschter Größe anzieht.

Für eine erfolgreiche Anzucht sind wichtig:

- eine gute Belüftung (aerober Organismus);
- ein Ausgangs-pH-Wert des Nährmediums zwischen 6,0 und 8,0;
- die Anwesenheit von N-Acetyl-L-prolin im Nährmedium zur Induktion des Enzyms (0,06 bis 0,1 Gewichtsprozent)
- die Anwesenheit von Vitaminen (z.B. in komplexer Form als Hefeextrakt).

Das Enzym kann nach Aufschluß der Zellen durch die Kombination an sich bekannter Methoden der Enzymreinigung gewonnen werden. Das Enzym kann zur Gewinnung von L-Prolin aus N-Acetyl-L-prolin, N-Chloracetyl-L-prolin, N-Acetyl-D,L-prolin oder N-Chloracetyl-D,L-prolin verwendet werden. Zudem kann das Enzym zur Herstellung von N-Acetyl-L-prolin, N-Propionyl-L-prolin und N-Butyryl-L-prolin aus L-Prolin und

der jeweiligen Carbonsäure eingesetzt werden.

Die Erfindung soll durch die nachfolgenden Beispiele näher erläutert werden:

Beispiel 1: Suche nach N-Acyl-L-prolin-Acylase-Produzenten

3 Boden- und 1 Klarwerksprobe wurden mit 0,9 % (w/v) NaCl aufgeschlemmt und 1 ml der Suspension bzw. 1 ml der unverdünnten Klärwerksprobe in 50 ml Flüsigmedium (Anreicherungsmedium) eingeimpft. Das Flüssigmedium hatte folgende Zusammensetzung:

| | |
|---|---|
| N-Acetyl-L-prolin | 5 g |
| Hefeextrakt | 0,1 g |
| $K_2HPO_4$ | 2 g |
| NaCl | 1 g |
| $MgSO_4 . 7H_2O$ | 0,3 g |
| $CaCl_2 . 2H_2O$ | 0,1 g |
| Spurenelement-Lösung | 1 ml |
| $H_2O$ demineralisiert ad | 1 l |
| pH-Wert | 5,6 bzw. 7,4 |

Die Spurenelement-Lösung hatte folgende Zusammensetzung:

| | |
|---|---|
| $FeCl_3 . 6H_2O$ | 250 mg |
| $ZnCl_2$ | 75 mg |
| $H_3BO_3$ | 30 mg |
| $CuSO_4 . 5H_2O$ | 20 mg |
| $MnCl_2 . 4H_2O$ | 20 mg |
| $CoCl_2 . 6H_2O$ | 14 mg |
| $(NH_4)_6Mo_7O_{24} . 4H_2O$ | 10 mg |
| 0,01 N HCl ad | 100 ml |
| pH-Wert | ~ 1,7 |

50 ml des Anreicherungsmediums wurden in 500 ml Erlenmeyerkolben (mit Aufsatz und 4 seitlichen Schikanen) gefüllt und autoklaviert. Die Spurenelementlösung wurde sterilfiltriert und jedem Kolben nach dem Abkühlen zugesetzt. Die Kolben wurden wie oben angegeben beimpft und bei 30° C aerob in einem Rundschüttler bei 200 rpm 3 Tage inkubiert. Dicht bewachsene Ansätze wurden mit steriler 0,9 % (w/v) NaCl-Lösung in üblicher Weise verdünnt und auf Agarplatten (Anreicherungsmedium mit 1,2 % (w/v) Agar-Agar, pH-Wert 5,6) ausplattiert. Die Agarplatten wurden 5 Tage bei 30° C inkubiert und gutgewachsene Kolonien vereinzelt und insgesamt 4- bis 5-mal auf das gleiche Medium überimpft.

Nach der Koloniemorphologie und mikroskopischem Bild einheitlich erscheinende Stämme wurden sodann in 100 ml Flüsigmedium (500 ml Erlenmeyerkolben mit Aufsatz und 4 seitlichen Schikanen) bei 30° auf einer Rundschüttelmaschine bei 200 rpm vermehrt. Das Anzuchtmedium hatte folgende Zusammensetzung:

| | |
|---|---|
| N-Acetyl-L-prolin | 4 g |
| $(NH_4)_2SO_4$ | 2 g |
| Hefeextrakt | 1 g |
| $K_2HPO_4$ | 2 g |
| $MgSO_4 . 7H_2O$ | 0,3 g |
| $CaCl_2 . 2H_2O$ | 0,1 g |
| NaCl | 1 g |
| Spurenelement-Lösung | 1 ml |
| $H_2O$ demineralisiert ad | 1 l |
| pH-Wert | 7,0 |

Nach 24 bis 48 Stunden wurde das zellhaltige Nährmedium zentrifugiert (15 min, 4.500 g in einer Kühlzentrifuge), die Zellen wurden zweimal mit 0,9 % (w/v) NaCl-Lösung gewaschen und in 10 ml 0,1 M Tris-HCl-Puffer, pH 7,0, resuspendiert.

Die Mikroorganismen dieser Suspension wurden durch Ultraschallbehandlung (Sonifier-Cell Disrupter B-30 der Firma Branson Sonic Power Co., Danbury, Connecticut, USA) aufgeschlossen (2 Minuten pulsierend, entsprechend 1 Minute reiner Beschalldauer). Die Zelltrümmer und nicht aufgeschlossene Zellen wurden in einer Kühlzentrifuge 30 Minuten bei 40.000 g und 5°C abzentrifugiert. Der klare Überstand (= Rohextrakt) wurden im Enzymtest eingesetzt.

Der Standard-Reaktionsansatz zur Bestimmung der Enzymaktivität war folgendermaßen zusammengesetzt:

| | | |
|---|---|---|
| 30 mM | N-Acetyl-L-prolin in 0,1 M Tris-HCl-Puffer, eingestellt auf pH 7,0 | 1,0 ml |
| 0,1 M | Tris-HCl-Puffer pH 7,0<br>Rohextrakt | 1,95 ml<br>0,05 ml |

Die Reaktion wurde durch Zugabe von Rohextrakt gestartet und die Reaktionsansätze üblicherweise 10 Minuten bei 30° C inkubiert. Inkubationszeit und Rohextraktmenge wurden so bemessen, daß der Linearitätsbereich der enzymatischen Reaktion und des nachgeschalteten Prolin-Nachweises nicht überschritten wurden.

Nachweis des Reaktionsproduktes L-Prolin durch Ninhydrin-Detektion (nach Yaron und Mlynar, Biochem. Biophys. Res. Commun. 32 (4), 658-663 (1968)).

50 µl des Enzymtests wurden in 1,25 ml 100%-ige Essigsäure pipettiert und damit die enzymatische Reaktion gestoppt. Anschließend wurden 0,45 ml 0,1 M Tris-HCl-Puffer pH 7,0 und 1,25 ml Ninhydrin-Reagenz (3 g Ninhydrin unter Erwärmen gelöst in einer Mischung aus 60 ml 100%-iger Essigsäure und 40 ml 6 M Phosphorsäure) zugegeben. Das Ninhydrin-Reagenz wurden täglich frisch angesetzt.

Die Reagenzgläser wurden mit Schraubdeckeln locker verschlossen, 30 Minuten bei 100° C inkubiert und anschließend im Eisbad abgekühlt.

Das durch die enzymatische Hydrolyse freigesetzte L-Prolin bildet mit Ninhydrin bei 100° C einen roten Farbstoff, dessen Absorption im Spektralphotometer bei 480 nm gegen einen Ansatz ohne Rohextrakt (= Substratblank) gemessen wurde. Anhand einer Eichgeraden mit L-Prolin im Bereich von 5 bis 100 nmole pro 50 µl Probe wurde die Konzentration des entstandenen L-Prolins bestimmt. Die Enzymaktivität wurde in internationalen Einheiten angegeben, wobei eine Einheit (U) einer Menge von 1 µmol freigesetztem L-Prolin pro Minute entsprach.

Die Proteinbestimmung wurde mit einem Proteinbestimmungs-Kit der Fa. Pierce Chemical Co., Rockford, Illinois, USA, nach Bradford, Anal. Biochem. 72, 248-254 (1976) durchgeführt.

Tabelle 1

| Bildung der N-Acyl-L-prolin-Acylase durch verschiedene gescreente Bakterienstämme | |
|---|---|
| Stamm | Spez. Aktivität (U/mg) |
| B-A1 (DSM 5417) | 0,64 |
| B-A2 | 0,34 |
| B-A3 | 0,55 |
| B-B | 0,59 |
| B-K (DSM 5416) | 2,98 |
| B-L | 0,19 |
| B-M | 0,09 |
| Sub 4 | 0,42 |
| Sub 5 | 0,16 |
| Sub 6 | 0,41 |

Wie Tabelle 1 zeigt, wies der Stamm B-K bei dem beschriebenen Testverfahren die weitaus höchste Aktivität auf und wurde daher zur Produktion des Enzyms verwendet. Die Stämme B-K und B-A1 wurden durch die Deutsche Sammlung von Mikroorganismen und Zellkulturen GmbH (DSM), Braunschweig, FRG, als Comamonas testosteroni und Alcaligenes denitrificans identifiziert.

Beispiel 2: Wachstum und Acylase-Bildung von Comamonas testosteroni DSM 5416.

a) Wachstum und Acylase-Bildung auf verschiedenen Kohlenstoffquellen

Comamonas testosteroni DSM 5416 wurde, wie in Beispiel 1 beschrieben, im Anzuchtmedium vermehrt, die Zellen durch Ultraschall aufgeschlossen und die spezifische Aktivität der N-Acyl-L-prolin-Acylase bestimmt. Die Kohlenstoffquelle des Anzuchtmediums mit einer Konzentration von 4 g/l wurde variiert und der Einfluß auf das Wachstum und die Acylase-Bildung bestimmt.

Tabelle 2

| Einfluß verschiedener Kohlenstoffquellen auf das Wachstum und die spezifische Aktivität der N-Acyl-L-prolin-Acylase | | |
|---|---|---|
| C-Quelle* | Wachstum ($OD_{600}$) | Spez. Aktivität (U/mg) |
| N-Acetyl-L-prolin | 6,0 | 2,26 |
| Acetat | 2,3 | < 0,01 |
| Propionat | 1,9 | < 0,01 |
| Butyrat | 1,8 | < 0,01 |
| Pyruvat | 2,4 | < 0,01 |
| Lactat | 3,0 | < 0,01 |
| Malat | 3,4 | < 0,01 |
| Glycerin | 1,1 | < 0,01 |
| L-Aspartat | 2,0 | < 0,01 |
| L-Leucin | 2,6 | < 0,01 |
| L-Prolin | 3,9 | < 0,01 |

* Kein Wachstum auf Arabinose, Glucose, Fructose, Lactose, Maltose, Mannose, Saccharose, Xylose, Citrat, Phenylacetat, L-Arginin, L-Histidin, L-Serin und L-Tryptophan

Wie aus Tabelle 2 zu ersehen ist, wurde die N-Acyl-L-prolin-Acylase durch Comamonas testosteroni DSM 5416 nur bei Wachstum auf N-Acetyl-L-prolin als Kohlenstoffquelle gebildet. Bei Wachstum auf anderen Kohlenstoffquellen war keine Enzym-Aktivität nachweisbar.

b) Wachstum und Acylase-Bildung bei verschiedenen Start-pH-Werten

Im Anzuchtmedium, wie in Beispiel 1 beschrieben, jedoch mit 2 g/l N-Acetyl-L-prolin, wurde der pH-Wert im Bereich 6,0 - 8,0 in Schritten zu 0,5 Einheiten variiert. Nach einer Inkubationszeit von 17,5 Stunden wurden der pH-Wert des Mediums, das Wachstum durch Messung der optischen Dichte bei 600 nm und die spezifische Aktivität der N-Acyl-L-prolin-Acylase bestimmt.

Tabelle 3

| Abhängigkeit des Wachstums und der Bildung der N-Acyl-L-prolin-Acylase vom pH-Wert des Anzuchtmediums | | | |
|---|---|---|---|
| Anfangs-pH | End-pH | Wachstum ($OD_{600}$) | Spez. Aktivität (U/mg) |
| 6,0 | 8,00 | 3,55 | 1,52 |
| 6,5 | 8,05 | 3,78 | 1,31 |
| 7,0 | 8,00 | 3,80 | 1,36 |
| 7,5 | 8,06 | 3,53 | 1,34 |
| 8,0 | 8,00 | 3,88 | 0,58 |

Tabelle 3 zeigt, daß der End-pH-Wert und das Wachstum durch den Ausgangs-pH-Wert des Anzuchtmediums nicht beeinflußt wurden. Im pH-Bereich 6,0 - 7,5 wird auch die spezifische Aktivität der Acylase nur unwesentlich beeinflußt, während bei pH 8,0 nur etwa ein Drittel der Enzym-Aktivität erzielt wird.

c) Wachstum und Acylase-Bildung bei verschiedenen N-Acetyl-L-prolin-Konzentrationen

Im Anzuchtmedium, wie in Beispiel 1 beschrieben, wurde die N-Acetyl-L-prolin-Konzentration im Bereich von 1 g/l - 5 g/l in Schritten zu 1 g/l variiert. Nach einer Inkubationszeit von 24 Stunden wurden das Wachstum und die spezifische Aktivität der N-Acyl-L-prolin-Acylase bestimmt.

Tabelle 4

| Abhängigkeit des Wachstums und der Bildung der N-Acyl-L-prolin-Acylase von der N-Acetyl-L-prolin-Konzentration des Anzuchtmediums | | |
|---|---|---|
| N-Acetyl-L-prolin (g/l) | Wachstum ($OD_{600}$) | Spez. Aktivität (U/mg) |
| ohne | 0,39 | n.b. |
| 1 | 1,95 | 1,33 |
| 2 | 3,16 | 1,36 |
| 3 | 4,32 | 1,77 |
| 4 | 5,42 | 1,84 |
| 5 | 5,68 | 0,94 |
| n.b. nicht bestimmt | | |

Tabelle 4 zeigt, daß die erzielte Zelldichte von der eingesetzten Menge N-Acetyl-L-prolin abhing. Ohne N-Acetyl-L-prolin ist nur geringes Wachstum, bedingt durch den im Anzuchtmedium vorhandenen Hefeextrakt, zu verzeichnen. Mit zunehmender N-Acetyl-L-prolin-Konzentration stieg die spezifische Aktivität der Acylase an und erreichte ein Optimum bei 4 g/l N-Acetyl-L-prolin.

d) Wachstum and Acylase-Bilding bei verschiedenen $(NH_4)_2SO_4$-Konzentrationen

Im Anzuchtmedium, wie in Beispiel 1 beschrieben, wurde die $(NH_4)_2SO_4$-Konzentration im Bereich von 1 g/l - 5 g/l in Schritten zu 1 g/l variiert. Nach einer Inkubationszeit von 23 Stunden wurden das Wachstum und die spezifische Aktivität der N-Acyl-L-prolin-Acylase bestimmt.

Tabelle 5

| Abhängigkeit des Wachstums und der Bildung der N-Acyl-L-prolin-Acylase von der $(NH_4)_2SO_4$-Konzentration des Anzuchtmediums | | |
|---|---|---|
| $(NH_4)_2SO_4$ (g/l) | Wachstum ($OD_{600}$) | Spez. Aktivität (U/mg) |
| ohne | 4,82 | 0,38 |
| 1 | 5,24 | 1,71 |
| 2 | 5,67 | 1,91 |
| 3 | 5,47 | 1,53 |
| 4 | 5,44 | 1,59 |
| 5 | 5,47 | 1,32 |

Tabelle 5 zeigt, daß das Wachstum und die Bildung der Acylase bei einer $(NH_4)_2SO_4$-Konzentration von 2 g/l optimal waren. $(NH_4)_2SO_4$ förderte offensichtlich die Bildung der Acylase, da ohne $(NH_4)_2SO_4$ die spezifische Aktivität der Acylase nur 20% des optimalen Wertes betrug, während die erreichte Zelldichte nur unwesentlich geringer lag. In diesem Fall dienten der im Anzuchtmedium vorhandene Hefeextrakt und das aus dem N-Acetyl-L-prolin freigesetzte L-Prolin als Stickstoffquelle für das Wachstum der Zellen.

e) Wachstum und Acylase-Bildung bei verschiedenen Hefeextrakt-Konzentrationen

Im Anzuchtmedium, wie in Beispiel 1 beschrieben, wurde die Hefeextrakt-Konzentration im Bereich von 1 g/l - 5 g/l in Schritten zu 1 g/l variiert. Nach einer Inkubationszeit von 24 Stunden wurden das Wachstum und die spezifische Aktivität der N-Acyl-L-prolin-Acylase bestimmt.

Tabelle 6

| Abhängigkeit des Wachstums und der Bildung der N-Acyl-L-prolin-Acylase von der Hefeextrakt-Konzentration des Anzuchtmediums | | |
|---|---|---|
| Hefeextrakt (g/l) | Wachstum ($OD_{600}$) | Spez. Aktivität (U/mg) |
| ohne | 0,02 | n.b. |
| 1 | 6,11 | 2,14 |
| 2 | 7,03 | 1,30 |
| 3 | 7,55 | 1,30 |
| 4 | 8,05 | 1,26 |
| 5 | 8,82 | 1,46 |
| n.b. nicht bestimmt | | |

Tabelle 6 zeigt, daß die Bildung der Acylase bei einer Hefextrakt-Konzentration von 1 g/l optimal war, während die erreichte Zelldichte mit zunehmender Hefeextrakt-Konzentration anstieg. Ohne Hefeextrakt wuchsen die Zellen nicht, was auf komplexe Nährstoffansprüche des Stammes deutet.

f) Wachstum und Acylase-Bildung bei gleichzeitigem Angebot von Acetat und N-Acetyl-L-prolin im Nährmedium

Im Anzuchtmedium, wie in Beispiel 1 beschrieben, wurde die N-Acetyl-L-prolin-Konzentration auf 0,2 bis 1,0 g/l reduziert und es wurden zusätzlich 4 g/l Na-Acetat eingesetzt. Nach einer Inkubationszeit von 34 Stunden wurden das Wachstum und die spezifische Aktivität der N-Acyl-L-prolin-Acylase bestimmt.

Tabelle 7

| Wachstum und Induktion der N-Acyl-L-prolin-Acylase bei gleichzeitigem Angebot von Na-Acetat und N-Acetyl-L-prolin im Nährmedium | | |
|---|---|---|
| N-Acetyl-L-prolin (g/l) | Wachstum ($OD_{600}$) | Spez. Aktivität (U/mg) |
| ohne | 2,35 | $< 0,01$ |
| 0,2 | 2,27 | 0,53 |
| 0,4 | 2,90 | 0,79 |
| 0,6 | 2,75 | 1,41 |
| 0,8 | 3,04 | 1,70 |
| 1,0 | 3,12 | 1,55 |

Tabelle 7 zeigt, daß die Acylase nur in Gegenwart von N-Acetyl-L-prolin gebildet wurde, gleichzeitig aber nicht durch Acetat reprimiert wurde. Diese erlaubt es durch gleichzeitiges Angebot von Acetat und N-Acetyl-L-prolin als Kohlenstoffquellen eine genügend große Menge an Biomasse zu erhalten, wobei 0,6 g/l des Induktors genügten, um die Bildung der Acylase ausreichend zu induzieren.

g) Wachstum und Acylase-Bildung bei Zugabe von N-Acetyl-L-prolin zum Nährmedium in der spätexponentiellen Wachstumsphase

Es wurde, wie oben unter f) beschrieben, verfahren. Der Induktor, N-Acetyl-L-prolin, wurde jedoch erst in der spätexponentiellen Wachstumsphase nach einer Inkubationszeit von 12 Stunden dem Nährmedium zugesetzt. Danach wurde das Nährmedium weitere 24 Stunden inkubiert und das Wachstum und die spezifische Aktivität der N-Acyl-L-prolin-Acylase bestimmt.

Tabelle 8

| Wachstum und Induktion der N-Acyl-L-prolin-Acylase bei Zugabe von N-Acetyl-L-prolin in der spätexponentiellen Wachstumsphase | | |
|---|---|---|
| N-Acetyl-L-prolin (g/l) | Wachstum ($OD_{600}$) | Spez. Aktivität (U/mg) |
| ohne | 1,92 | < 0,01 |
| 0,2 | 2,22 | 0,50 |
| 0,4 | 2,28 | 0,63 |
| 0,6 | 2,76 | 0,96 |
| 0,8 | 2,95 | 0,67 |
| 1,0 | 2,88 | 1,08 |

Ein Vergleich der Tabellen 7 und 8 zeigt, daß in einem Acetat-haltigen Nährmedium 0,6 g/l N-Acetyl-L-prolin ausreichend waren, um die Acylase-Bildung ausreichend zu induzieren. Hierbei ist es vorteilhaft, den Induktor bereits vor dem Animpfen dem Nährmedium zuzusetzen.

h) Wachstum und Acylase-Bildung bei gleichzeitigem Angebot von Na-Acetat und verschiedenen N-Acetyl- und N-Chloracetyl-aminosäuren im Nährmedium

Es wurde, wie oben unter f) beschrieben, verfahren. Neben N-Acetyl-L-prolin wurden verschiedene N-Acetyl- und N-Chloracetyl-aminosäuren in einer Konzentration von 1 g/l im Nährmedium eingesetzt. Nach einer Inkubationszeit von 24 Stunden wurden das Wachstum und die spezifische Aktivität der N-Acyl-L-prolin-Acylase bestimmt.

Tabelle 9

| Einfluß verschiedener N-Acetyl- und N-Chloracetyl-aminosäuren auf das Wachstum und die Acylase-Bildung | | |
|---|---|---|
| Induktor | Wachstum ($OD_{600}$) | Spez. Aktivität (U/mg) |
| ohne | 2,60 | < 0,01 |
| Ac-L-Ala | 2,28 | < 0,01 |
| Ac-L-Pro | 3,46 | 1,34 |
| ClAc-L-Met | 2,19 | < 0,01 |
| ClAc-L-Val | 2,50 | < 0,01 |
| ClAc-L-Leu | 2,65 | < 0,01 |
| ClAc-L-Phe | 2,89 | < 0,01 |
| ClAc-L-Tyr | 2,76 | < 0,01 |

Wie aus Tabelle 9 zu ersehen ist, induzierte unter den getesteten N-Acetyl- und N-Chloracetyl-aminosäuren ausschließlich N-Acetyl-L-prolin die Acylase-Bildung.

Beispiel 3: Reinigung der N-Acyl-L-prolin-Acylase

a) Anzucht von Comamonas testosteroni DSM 5416 und Gewinnung des Rohextraktes

Comamonas testosteroni DSM 5416 wurde auf Schrägagar (Caseinpepton-Sojamehlpepton-Agar der Firma Merck AG, Darmstadt, FRG) angezogen und je Schrägagar-Röhrchen mit 4,5 ml steriler 0,9 % (w/v) NaCl-Lösung abgeschwemmt. Die so erhaltene Zellsuspension diente als Inokulum für 4 Erlenmeyerkolben (2 l mit 4 Schikanen), die jeweils 500 ml Anzuchtmedium, wie in Beispiel 1 beschrieben, enthielten. Die Erlenmeyerkolben wurden 21 Stunden auf einem Rundschüttler bei 30° C und 100 rpm inkubiert und die Zellen, wie im Beispiel 1 beschrieben, geerntet. gewaschen, in 30 ml 0,1 M Tris-HCl-Puffer pH 7,0 resuspendiert und durch Ultraschall aufgeschlossen.

b) 70° C - Hitzefällung

Der zellfreie Rohextrakt (38,5 ml) wurde 30 Minuten im Wasserbad bei einer Temperatur von 70° C inkubiert. Das nach dieser Zeit präzipitierte Protein wurde durch Zentrifugation (30 Minuten bei 40.000 g und 5° C) abgetrennt.

c) Konzentrierung durch Filtration

Der Überstand der 70° C-Hitzefällung (34.5 ml) wurde durch Filtration über eine Flachmembran der Ausschlußgrenze 100.000 Dalton (YM 100) in einer Rührzelle (Modell 8050 der Firma W.R. Grace Co., Amicon Division, Danvers, USA) bei einem Druck von 4 bar unter Stickstoff konzentriert.

d) Fast Protein Liquid Chromatography (FPLC) an Mono Q

Das Retentat der Filtration (4,5 ml) wurde auf eine 0,5 x 5 cm Mono Q-Säule (Firma Pharmacia / LKB, Uppsala, Schweden) aufgetragen und bei einem Flow von 1 ml/min chromatographiert. Der Anionenaustauscher wurde vor der Probenaufgabe mit einem 0,1 M Tris-HCl-Puffer pH 7,0 äquilibriert. Eluiert wurde mit einem linearen, innerhalb von 20 ml von 0 auf 0,4 M ansteigenden NaCl-Gradienten in 0,1 M Tris-HCl-Puffer pH 7,0. Die Elution des Enzyms erfolgte bei 0,2 - 0,25 M NaCl. Es wurden, bei einem Probevolumen von 0,2 ml je Lauf, insgesamt 14 Läufe durchgeführt. Die aktiven Fraktionen wurden vereinigt und bei -20° C eingefroren.

Die Ergebnisse der Aufreinigung zeigt Tabelle 10.

Tabelle 10: Reinigungsschema der N-Acyl-L-prolin-Acylase

| Reinigungsschritt | Volumen (mL) | Gesamt-Protein (mg) | Gesamt-Aktivität (U) | Ausbeute (%) | Spez.Aktivität (U/mg) | Anreicherung (-fach) |
|---|---|---|---|---|---|---|
| Rohextrakt | 38,5 | 472,0 | 754,7 | 100 | 1,60 | 1 |
| 70°C-Hitzefällung | 34,5 | 86,6 | 618,0 | 82 | 7,14 | 4,5 |
| Konzentrierung (Amicon-Rührzelle mit YM-100-Membran, 100.000 D) | 4,5 | 56,2 | 585,6 | 78 | 10,42 | 6,5 |
| FPLC-Mono Q | 27,5 | 4,4 | 374,7 | 50 | 85,16 | 53,2 |

Beispiel 4: Abhängigkeit der Reaktionsgeschwindigkeit vom pH-Wert

Die Reaktionsgeschwindigkeit der hydrolytischen Abspaltung von Essigsäure aus der Verbindung N-Acetyl-L-prolin in Gegenwart der N-Acyl-L-prolin-Acylase wurde in Abhängigkeit vom pH-Wert des Reaktionsgemisches bestimmt. Der Testansatz hatte folgende Zusammensetzung:

| | | |
|---|---|---|
| 10 mM | N-Acetyl-L-prolin in dem unten angegebenen 0,1 M Puffer<br>Acylase | 2,95 ml<br>0,05 ml |

Vor dem Start der Reaktion durch Acylase-Zugabe wurden die pH-Werte des Substrat-Puffer-Gemisches im Bereich von 4,0 bis 5,0 in 0,1 M Essigsäure/NaOH-Puffer, im Bereich von 5,0 bis 6,0 in 0,1 M Na-Citrat/NaOH-Puffer, im Bereich von 6,0 bis 7,5 in 0,1 M $K_2HPO_4$ / $KH_2PO_4$-Puffer, im Bereich von 6,2 bis 9,0 in 0,1 M Tris-HCl-Puffer und im Bereich von 9,0 bis 10,0 in 0,1 M $Na_2CO_3$ / $NaHCO_3$-Puffer durch Zugabe von 2 N NaOH auf dem gewünschten pH-Wert eingestellt. Nach 10 Minuten Reaktionszeit bei 30° C wurde die Enzymaktivität durch Ninhydrin-Nachweis des gebildeten L-Prolins bestimmt.

Das Optimum der Reaktionsgeschwindigkeit im Tris-HCl-Puffer lag im pH-Bereich zwischen 6,2 und 7,2, im Kaliumphosphat-Puffer lag das pH-Optimum im Bereich > 7,5, die Reaktionsgeschwindigkeit betrug jedoch hier im Vergleich zum Tris-HCl-Puffer weniger als 15 %. Phosphat hemmte das Enzym.

Beispiel 5: Optimale Reaktionstemperatur

Reaktionsansätze mit 2,95 ml 10 mM N-Acetyl-L-prolin in 0,1 M Tris-HCl-Puffer pH 7,5 wurden 10 Minuten bei Temperaturen zwischen 22 und 85° C vortemperiert und dann die Reaktion durch Zugabe von 0,05 ml Acylase gestartet. Nach 5 Minuten Reaktionszeit wurde die Enzymaktivität durch Ninhydrin-Nachweis des gebildeten L-Prolins bestimmt.

Die maximale Reaktionsgeschwindigkeit wurde bei 65° C erreicht und war um den Faktor 2,3 höher als bei der Standardtemperatur von 30° C.

Beispiel 6: Stabilität der N-Acyl-L-prolin-Acylase

a) pH-Stabilität

Die pH-Stabilität der N-Acyl-L-prolin-Acylase wurde im pH-Bereich von 4,2 bis 10,4 untersucht. FPLC/Mono Q gereinigtes Enzym wurde mit verschiedenen 0,1 M Puffern unterschiedlicher pH-Werte 10-fach verdünnt und 3 Wochen bei 22° C gelagert. Zu verschiedenen Zeiten wurden Proben entnommen (0,2 ml) und deren Enzymaktivität bei 30° C im Reaktionsansatz folgender Zusammensetzung bestimmt:

| | | |
|---|---|---|
| 10 mM | N-Acetyl-L-prolin in 0,1 M Tris-HCl-Puffer pH 7,5<br>Acylase (1 : 10 verdünnt) | 2,8 ml<br>0,2 ml |

Wie aus Tabelle 11 ersichtlich ist, wurde das Enzym durch Citrat rasch inaktiviert, während bei Lagerung in Tris-HCl-Puffer und Na-Carbonat-Puffer zwischen pH 7,4 und 10,4 kein nennenswerter Aktivitätsverlust feststellbar war.

Tabelle 11

| pH-Stabilität der N-Acyl-L-prolin-Acylase | | | | | |
|---|---|---|---|---|---|
| Puffer | Restaktivität ( % ) nach | | | | |
| | 1 Tag | 3 Tagen | 1 Woche | 2 Wochen | 3 Wochen |
| Na-Acetat | | | | | |
| pH 4,2 | 65 | 56 | 34 | 22 | 16 |
| pH 4,6 | 59 | 58 | 49 | 38 | 28 |
| pH 5,2 | 73 | 75 | 64 | 71 | 81 |
| Na-Citrat | | | | | |
| pH 5,0 | 14 | 1 | 0 | 0 | 0 |
| pH 5,5 | 16 | 3 | 1 | 0 | 0 |
| pH 6,0 | 21 | 5 | 3 | 5 | 3 |
| K-Phosphat | | | | | |
| pH 6,0 | 82 | 96 | 68 | 48 | 30 |
| pH 6,5 | 84 | 96 | 67 | 52 | 40 |
| pH 7,0 | 86 | 97 | 72 | 63 | 49 |
| pH 7,5 | 88 | 98 | 69 | 56 | 48 |
| Tris-HCl | | | | | |
| pH 7,4 | 82 | 101 | 97 | 145 | 156 |
| pH 8,0 | 107 | 110 | 107 | 163 | 171 |
| pH 8,5 | 117 | 129 | 123 | 166 | 162 |
| pH 9,0 | 101 | 128 | 122 | 160 | 1 |
| Na-Carbonat | | | | | |
| pH 8,7 | 93 | 109 | 101 | 133 | 127 |
| pH 9,3 | 93 | 101 | 98 | 65 | 38 |
| pH 9,9 | 98 | 107 | 102 | 134 | 108 |
| pH 10,4 | 121 | 132 | 115 | 128 | 111 |

b) Temperaturstabilität

Die in 0,1 M Tris-HCl-Puffer pH 7,5 gelöste Acylase wurde 30 Minuten bei Temperaturen von 20 bis 85° C inkubiert. Danach wurde die Enzymaktivität bei 30° C im Reaktionsansatz folgender Zusammensetzung bestimmt:

| | | |
|---|---|---|
| 10 mM | N-Acetyl-L-prolin, in 0,1 M Tris-HCl-Puffer pH 7,5<br>Acylase | 2,95 ml<br>0,05 ml |

Nach 30-minütiger Inkubation bei 70° C war kein Aktivitätsverlust des Enzyms nachweisbar, bei höheren Temperaturen wurde das Enzym jedoch schnell desaktiviert.

Beispiel 7: Einflüsse von Inhibitoren und Aktivatoren

Der Einfluß verschiedener chemischer Agenzien sowie Metallkationen und Anionen auf die Reaktionsgeschwindigkeit der hydrolytischen Spaltung von N-Acetyl-L-prolin wurde bei 30° C im Reaktionsansatz folgender Zusammensetzung bestimmt:

| | | |
|---|---|---|
| 30 mM<br>3 bzw. 30 mM | N-Acetyl-L-prolin, in 0,1 M Tris-HCl-Puffer pH 7,0<br>Inhibitor in o.g. Puffer | 1,0 ml<br>1,0 ml |
| 0,1 M | Tris-HCl-Puffer pH 7,0<br>Acylase | 0,95 ml<br>0,05 ml |

a) Einfluß verschiedener chemischer Agenzien

Aus Tabelle 12 geht hervor, daß 1,10-Phenanthrolin, 2-Mercaptoethanol, 4-Chloromercuribenzoat und 4-Hydroxymercuribenzoat die Acylase signifikant hemmen. Vor dem Start der Reaktion durch Substratzugabe wurde das Enzym 10 Minuten bei 30° C mit dem Effektor vorinkubiert.

Tabelle 12

| Einfluß verschiedener chemicher Agenzien auf die Acylase-Aktivität | | |
|---|---|---|
| Effektor | Konzentration (mM) | Aktivität (%) |
| ohne | - | 100 |
| EDTA | 1<br>10 | 101<br>99 |
| Na-Citrat | 1<br>10 | 102<br>102 |
| 2,2'-Bipyridin | 1<br>10 | 102<br>66 |
| 1,10-Phenanthrolin | 1<br>10 | 27<br>1 |
| Dithiothreitol | 1<br>10 | 129<br>48 |
| Glutathion | 1<br>10 | 116<br>135 |
| 2-Mercaptoethanol | 1.4<br>14 | 42<br>14 |
| 4-Chlormercuribenzoat | 1<br>10 | 85<br>48 |
| 4-Hydroxymercuribenzoat | 1<br>10 | 88<br>41 |
| Jodacetamid | 1<br>10 | 104<br>101 |
| Jodacetat | 1<br>10 | 97<br>77 |
| Semicarbazid | 1<br>10 | 109<br>95 |
| PMSF | 0.1<br>1 | 104<br>130 |

b) Einfluß verschiedener Metallkationen

Aus Tabelle 13 geht hervor, daß das native Enzym durch keines der getesteten Metallkationen aktiviert wurde. Inhibierend wirkten $Cd^{2+}$, $Cu^{2+}$, $Fe^{2+}$, $Fe^{3+}$, $Hg^{2+}$, $Sn^{2+}$ und $Zn^{2+}$. Vor dem Start der Reaktion durch Substratzugabe wurde das Enzym 10 Minuten bei 30° C mit dem Effektor vorinkubiert.

Tabelle 13

| Einfluß verschiedener Kationen auf die Acylase-Aktivität | | |
|---|---|---|
| Effektor | Konzentration (mM) | Aktivität (%) |
| ohne | - | 100 |
| $BaCl_2$ | 1<br>10 | 100<br>99 |
| $CaCl_2$ | 1<br>10 | 100<br>91 |
| $CdCl_2$ | 1<br>10 | 89<br>65 |
| $CoCl_2$ | 1<br>10 | 103<br>92 |
| $CuSO_4$ | 1<br>10 | 61<br>19 |
| $FeSO_4$ | 1<br>10 | 20<br>n.b. |
| $FeCl_3$ | 1<br>10 | 83<br>58 |
| $HgCl_2$ | 1<br>10 | 67<br>9 |
| $MgCl_2$ | 1<br>10 | 106<br>99 |
| $MnCl_2$ | 1<br>10 | 104<br>103 |
| $Ni(NO_3)_2$ | 1<br>10 | 94<br>75 |
| $SnCl_2$ | 1<br>10 | 72<br>55 |
| $SrCl_2$ | 1<br>10 | 104<br>100 |
| $ZnSO_4$ | 1<br>10 | 77<br>32 |
| n.b. nicht bestimmt | | |

c) Einfluß verschiedener Anionen

Aus Tabelle 14 geht hervor, daß die Acylase durch Phosphat stark und durch Carbonat und Nitrit schwach gehemmt wurde. Der Hemmechanismus des Phosphats war nicht-kompetitiv. Vor dem Start der Reaktion durch Substratzugabe wurde das Enzym 10 Minuten bei 30° C mit dem Effektor vorinkubiert. Die Konzentration der Anionen betrug jeweils 10 mM.

Tabelle 14

| Einfluß verschiedener Anionen auf die Acylase-Aktivität | |
|---|---|
| Effektor | Aktivität (%) |
| ohne | 100 |
| NaCl | 99 |
| $Na_2CO_3$ | 75 |
| $NaH_2PO_4$ | 6 |
| $Na_2SO_4$ | 96 |
| $NaNO_2$ | 71 |
| $KNO_3$ | 96 |

d) Einfluß verschiedener Kationen auf die 1,10-Phenanthrolin-inaktivierte Acylase (Apoenzym)

FPLC/Mono Q-gereinigtes Enzym wurde 24 Stunden bei 4° C in Anwesenheit von 1 mM 1,10-Phenanthrolin inkubiert. Anschließend wurden Enzym und der Chelatbildner 1,10-Phenanthrolin durch Gelfiltration über Sephadex G-25 M (PD-10, Firma Pharmacia / LKB, Uppsala, Schweden) getrennt. Die enzymhaltigen Fraktionen wurden vereinigt und die Aktivität ohne und in Anwesenheit verschiedener Kationen (1 mM) bestimmt.

Tabelle 15 zeigt, daß die 1,10-Phenanthrolin-inaktivierte Acylase (Apoenzym) durch $CO^{2+}$ und $Zn^{2+}$ auf 80 % bzw. 54 % der Ausgangsaktivität reaktiviert wurde. Wie bereits unter b) beschrieben, hemmten $Cu^{2+}$, $Fe^{2+}$, $Fe^{3+}$, $Hg^{2+}$ und $Sn^{2+}$. Dies deutet daraufhin, daß es sich bei der N-Acyl-L-prolin-Acylase um ein Kobalt- oder Zink-abhängiges Metalloenzym handelt.

Tabelle 15

| Einfluß verschiedener Kationen auf die 1,10-Phenanthrolin-inaktivierteAcylase | | |
|---|---|---|
| Enzym-Präparat | Effektor | Aktivität (%) |
| Natives Enzym | - | 100 |
| Inaktiviertes Enzym (vor Gelfiltration) | - | 2 |
| Inaktiviertes Enzym (nach Gelfiltration) | ohne | 21 |
| | $BaCl_2$ | 18 |
| | $CaCl_2$ | 17 |
| | $CdCl_2$ | 20 |
| | $CoCl_2$ | 80 |
| | $CuSO_4$ | 6 |
| | $FeSO_4$ | 12 |
| | $FeCl_3$ | 5 |
| | $HgCl_2$ | 6 |
| | $MgCl_2$ | 25 |
| | $MnCl_2$ | 23 |
| | $Ni(NO_3)_2$ | 21 |
| | $SnCl_2$ | 10 |
| | $SrCl_2$ | 23 |
| | $ZnSO_4$ | 54 |

Beispiel 8: Bestimmung des Molekulargewichts und der Anzahl und Größe der Untereinheiten

Das Molekulargewicht des nativen Enzyms wurde durch Gelfiltration an Superose 12 HR 10/30 ermittelt. Die an ein FPLC-System (Firma Pharmacia/LKB, Uppsala, Schweden) gekoppelte Säule (1,0 x 30 cm) wurde mit einer Durchflußrate von 0,3 ml pro Minute betrieben, wobei 0,2 ml des FPLC/Mono Q-gereinigten Enzyms als Probe dienten. Als Eichproteine wurden Aprotinin, Chymotrypsinogen A, Aldolase, Catalase und

Ferritin verwendet. Das Molakulargewicht der N-Acyl-L-prolin-Acylase beträgt 380.000 ± 40.000 Dalton.

Durch Gelelektrophorese in Gegenwart von Natriumdodecylsulfat (SDS) wurde die Größe und Anzahl der Untereinheiten des Enzyms bestimmt. Das Molekulargewicht der Untereinheiten beträgt 45.000 ± 5.000 Dalton. Das bedeutet, daß die N-Acyl-L-prolin-Acylase aus 8 Untereinheiten identischer Größe besteht. Für die Eichkurve wurden Phosphorylase b (Kaninchenmuskel), Albumin (Rinderserum), Ovalbumin (Hühnereiweiß), Carboanhydrase (Rinder-Erythrozyten) und Trypsin-Inhibitor (Sojabohne) verwendet.

Beispiel 9: Abhängigkeit der Acylase-Aktivität von der Substratkonzentration

Die Abhängigkeit der Reaktionsgeschwindigkeit der hydrolytischen Abspaltung von Essigsäure aus der Verbindung N-Acetyl-L-prolin in Gegenwart der N-Acyl-L-prolin-Acylase wurde im Reaktionsansatz folgender Zusammensetzung bestimmt:

| | |
|---|---|
| N-Acetyl-L-prolin in 0,1 M Tris-HCl-Puffer pH 7,0 | 1,0 ml |
| 0,1 M Tris-HCl-Puffer pH 7,0 | 1,95 ml |
| Acylase | 0,05 ml |

Die Reaktionstemperatur betrug 30° C und die Inkubationszeit 10 Minuten. Die N-Acetyl-L-prolin-Konzentration des Reaktionsansatzes wurde im Bereich von 1 bis 50 mM variiert. Der $K_M$-Wertfür N-Acetyl-L-prolin beträgt unter den o.g. Bedingungen 5 mM.

Beispiel 10: Substratspezifität der N-Acyl-L-prolin-Acylase

a) Hydrolyse verschiedener N-Acetyl-L-aminosäuren bzw. N-Chloracetyl-L-aminosäuren

Die Aktivität der Acylase wurde mit verschiedenen N-Acetyl-L-aminosäuren bzw. N-Chloracetyl-L-aminosäuren im Reaktionsansatz folgender Zusammensetzung bestimmt:

| | | |
|---|---|---|
| 20 mM | N-Acetyl-aminosäure bzw. N-Chloracetyl-aminosäure | 1,0 ml |
| 0,1 M | Tris-HCl-Puffer pH 7,0<br>Acylase | 0,95 ml<br>0,05 ml |

Die Reaktionstemperatur betrug 30°C und die Inkubationszeit 15 Minuten bis 24 Stunden. Jeweils 0,2 ml des Reaktionsansatzes wurden zu verschiedenen Zeiten mit 0,2 ml 10 % (w/v) Trichloressigsäure versetzt und das denaturierte Protein 10 Minuten bei 11.000 rpm in einer Tischzentrifuge abzentrifugiert. Der Überstand wurde mit einem 0,1 M Na-Citrat-Puffer pH 2,2 mit 25 % (v/v) 2,2-Thiodiethanol und 0,1 % (v/v) Phenol 1 : 5 bzw. 1 : 50 verdünnt. Die freigesetzten Aminosäuren wurden durch einen Aminosäurenanalysator LC 5001 der Firma Biotronic, Maintal, FRG, quantitativ erfaßt. Die in Tabelle 16 mit * gekennzeichneten Substrate und entsprechenden Produkte wurden durch HPLC quantifiziert.

Für die Bestimmung der relativen Aktivitäten wurde die Hydrolyserate mit N-Acetyl-L-prolin unter vergleichbaren Reaktionsbedingungen gleich 100 % gesetzt.

Wie aus Tabelle 16 zu ersehen ist, werden N-Acetyl- und N-Chloracetyl-L-prolin, N-Chloracetyl-L-thiazolidin-4-carbonsäure sowie N-Chloracetyl-DL-thiazolidin-2-carbonsäure bevorzugt hydrolysiert. Daneben wurden auch N-Chloracetyl-D,L-pipecolinsäure, N-Chloracetyl-L-methionin, N-Chloracetyl-L-valin sowie N-Acetyl-L-alanin mit deutlich geringerer Geschwindigkeit umgesetzt.

Tabelle 16

| Spezifität der N-Acyl-L-prolin-Acylase gegenüber verschiedenen N-Acetyl- und N-Chloracetyl-aminosäuren | |
|---|---|
| Substrat | Rel. Aktivität (%) |
| Ac-L-Pro | 100 |
| Ac-L-Ala | 9,3 |
| Ac-L-Val | 0,2 |
| Ac-D,L-Ser | 0,2 |
| Ac-L-Cys | 0 |
| Ac-L-Tyr | 0 |
| ClAc-L-Pro | 362 |
| ClAc-L-thiazolidin-4-carbonsäure | 462 |
| ClAc-D,L-thiazolidin-2-carbonsäure*+ | 202 |
| ClAc-D,L-pipecolinsäure*+ | 16 |
| Ac-L-azetidin-2-carbonsäure* | 0 |
| ClAc-L-indolin-2-carbonsäure* | 0 |
| ClAc-L-Met | 17,1 |
| ClAc-L-Val | 14,1 |
| ClAc-L-Leu | 1,9 |
| ClAc-L-Phe | 1,0 |
| ClAc-L-Tyr | 1,0 |
| ClAc-L-Ile | 0,5 |
| ClAc-D,L-1-aminocyclohexansäure | 0 |

+ Die Substratkonzentration betrug 50 mM.

b) Hydrolyse verschiedener Prolinderivate

Die Aktivität der Acylase wurde mit verschiedenen Prolinderivaten wie unter a) beschrieben bestimmt.

Für die Bestimmung der relativen Aktivitäten wurde die Hydrolyserate mit N-Acetyl-L-prolin unter vergleichbaren Reaktionsbedingungen gleich 100 % gesetzt.

Wie aus Tabelle 17 zu ersehen ist, ist die Acylase L-spezifisch, da N-Acetyl-D-Prolin nicht hydrolysiert wird. Das Enzym benötigt offensichtlich die freie Carboxylgruppe des Prolins zur Substratbindung, da N-Acetyl-L-prolinamid und N-Acetyl-L-prolinmethylester nicht hydrolysiert werden. N-Chloracetyl-L-prolin wurde um das 3,6-fache schneller hydrolysiert als N-Acetyl-L-prolin. Eine um das 3- bis 20-fach geringere Hydrolyserate wurde mit N-Formyl-L-prolin, N-Propionyl-L-prolin, N-Butyryl-L-prolin, N-Valeryl-L-prolin, N-Caproyl-L-prolin, N-Acetyl-L-4-hydroxyprolin, N-Benzyloxycarbonyl-glycyl-L-prolin und Gycyl-L-prolin erhalten.

Tabelle 17

| Spezifität der N-Acyl-L-prolin-Acylase gegenüber verschiedenen Prolinderivaten | |
|---|---|
| Substrat | Rel. Aktivität (%) |
| Ac-L-Pro-OH | 100 |
| Ac-D-Pro-OH | 0 |
| ClAc-L-Pro-OH | 362 |
| Ac-L-Pro-$NH_2$ | 0 |
| Ac-L-Pro-OMe | 0 |
| Ac-L-Pro(4-OH)-OH | 10 |
| Ac-L-Ala-L-Pro-OH | 0 |
| Formyl-L-Pro-OH | 18 |
| Propionyl-L-Pro-OH | 29 |
| Butyryl-L-Pro-OH | 14 |
| Valeryl-L-Pro-OH | 15 |
| Caproyl-L-Pro-OH | 9 |
| Z-L-Pro-OH | 0 |
| Z-D-Pro-OH | 0 |
| Z-L-Pro-$NH_2$ | 0 |
| Z-L-Pro-L-Ala-OH | 0 |
| Z-Gly-L-Pro-OH | 11 |
| Boc-L-Pro-OH | 0 |
| H-Gly-L-Pro-OH | 4 |
| H-L-Ala-L-Pro-OH | 0 |
| H-L-Pro-$NH_2$ | 0 |
| H-L-Pro-Gly-OH | 0 |

Beispiel 11: Stereospezifität der Hydrolyse von N-Acetyl-D,L-Prolin durch die N-Acyl-L-prolin-Acylase

Die Hydrolyse von N-Acetyl-L-prolin, N-Acetyl-D,L-prolin und N-Acetyl-D-prolin wurde im Reaktionsansatz folgender Zusammensetzung durchgeführt:

| | | |
|---|---|---|
| 20 mM | Substrat in 0,1 M Tris-HCl-Puffer pH 7,0 | 1,0 ml |
| 0,1 M | Tris-HCl-Puffer pH 7,0<br>Acylase | 0,95 ml<br>0,05 ml |

Der Reaktionsansatz wurde 2 Stunden bei 30°C inkubiert und dann die Menge gebildetes L-Prolin durch Aminosäureanalyse, wie im Beispiel 10 beschrieben, bestimmt.

Die Tabelle 18 zeigt, daß die N-Acyl-L-prolin-Acylase N-Acetyl-L-prolin, jedoch nicht N-Acetyl-D-prolin, hydrolysiert.

Tabelle 18

| Stereospezifität der N-Acyl-L-prolin-Acylase | | |
|---|---|---|
| Substrat | Konzentration (mmol/l) | L-Prolin (mmol/l) |
| N-Acetyl-L-prolin | 10 | 10,25 |
| N-Acetyl-D,L-prolin | 10 | 4,82 |
| N-Acetyl-D-prolin | 10 | $< 0,01$ |

Beispiel 12: Synthese von N-Acetyl-L-prolin, N-Propionyl-L-prolin und N-Butyryl-L-prolin

Die Rückreaktion, die Synthese von N-Acetyl-L-prolin aus Essigsäure und L-Prolin, wurde im Reaktionsansatz folgender Zusammensetzung untersucht:

| | | |
|---|---|---|
| 1 M | Natriumacetat + 5 M L-Prolin in 0,1 M Tris-HCl-Puffer pH 7,0<br>Acylase (FPLC-gereinigt) | 1 ml<br>1 ml |

Der Reaktionsansatz wurde bei 30° C inkubiert. Zu verschiedenen Zeiten wurden Proben entnommen, mit einem Aliquot 10 % (w/v) Trichloressigsäure versetzt und das denaturierte Protein 10 Minuten bei 11.000 rpm in einer Tischzentrifuge abzentrifugiert. Der Überstand wurde durch HPLC nach entsprechender Verdünnung mit dem Eluenten analysiert.

Nach einer Inkubationszeit von 7 Tagen betrug die N-Acetyl-L-prolin-Konzentration des Reaktionsansatzes 0,2 Mol/l, entsprechend einer Ausbeute von 40 %, bezogen auf die eingesetzte Mengen an Natriumacetat. Der Kontrollansatz ohne Enzym enthielt kein N-Acetyl-L-prolin.

Analog konnten auch N-Propionyl-L-prolin und N-Butyryl-L-prolin aus Na-Propionat bzw. Na-Butyrat und L-Prolin synthetisiert werden. Eine Synthese von N-Benzoyl-L-prolin, N-Phenylacetyl-L-prolin und N-Phenylpropionyl-L-prolin aus Natrium-Benzoat, -Phenylacetat bzw. -Phenylpropionat (jeweils 0,05 M im Reaktionsansatz) und L-Prolin gelang nicht.

**Patentansprüche**

**1.** Mikrobiologisch hergestellt
N-Acyl-L-prolin-Acylase,
gekennzeichnet durch die folgenden Eigenschaften:
1) Reaktivität:
sie spaltet die Acetylgruppe von N-Acetyl-L-prolin ab, wobei Essigsäure und L-Prolin als Endprodukte entstehen, und kondensiert Essigsäure und L-Prolin, wobei N-Acetyl-L-prolin und Wasser als Endprodukte entstehen;
2) Substratspezifität:
sie hydrolysiert N-Acetyl-L-prolin,
N-Chloracetyl-L-prolin,
N-Formyl-L-prolin,
N-Propionyl-L-prolin,
N-Butyryl-L-prolin,
N-Valeryl-L-prolin,
N-Caproyl-L-prolin,
N-Acetyl-L-4-hydroxyprolin,
N-Chloracetyl-L-thiazolidin-4-carbonsäure,
N-Chloracetyl-L-thiazolidin-2-carbonsäure,
N-Chloracetyl-L-pipecolinsäure,
N-Benzyloxycarbonyl-glycyl-L-prolin,
Glycyl-L-prolin, N-Acetyl-L-alanin,
N-Chloracetyl-L-methionin und
N-Chloracetyl-L-valin;
3) Optimaler pH-Wert:
der optimale pH-Wert ist 6,8 ± 0,5;
4) pH-Stabilität:
sie zeigt bei 22°C über einen Zeitraum von 3 Wochen eine gute Stabilität im pH-Bereich zwischen pH 7,0 und pH 10,0;
5) Optimale Temperatur:
die optimale Temperatur beträgt 65°C bei einem pH-Wert von 7,5;
6) Temperaturbeständigkeit:
bei 70°C und pH 7,5 ist nach 30-minütiger Inkubation kein Aktivitätsverlust nachweisbar;
7) Einflüsse von Inhibitoren und Aktivatoren:
inhibierend wirken von allem 1,10-Phenanthrolin, 2-Mercaptoethanol, 4-Chloromercuribenzoat, 4-Hydroxymercuribenzoat, $Hg^{2+}$, $Cu^{2+}$, $Fe^{2+}$, $Fe^{3+}$, $Sn^{2+}$, $Zn^{2+}$ und $PO_4^{3-}$, aktivierend auf das Apoen-

zym wirken $Co^{2+}$ und $Zn^{2+}$;
8) Molekulargewicht:
das Molekulargewicht beträgt 380 000 ± 40 000 Dalton;
9) Untereinheiten:
das Molekül besteht aus 8 gleichgroßen Untereinheiten mit je 45 000 ± 5 000 Dalton;
10) $K_M$-Wert:
der $K_M$-Wert für das Substrat N-Acetyl-L-prolin beträgt 5 mM (30°C, 0,1 M Tris-HCl-Puffer, pH 7,0).

**2.** Verfahren zur Gewinnung der N-Acyl-L-prolin-Acylase gemäß Anspruch 1,
dadurch gekennzeichnet,
daß man Comamonas testosteroni DSM 5416 oder Alkaligenes denitrificans DSM 5417 in einem wäßrigen Nährmedium, das eine Quelle für Kohlenstoff und Stickstoff, Mineralsalze und N-Acetyl-L-prolin als Induktor, bei Einsatz von Comamonas testosteroni DSM 5416 zusätzlich eine Vitaminquelle, z.B. in komplexer Form als hefeextrakt, enthält, bei einem Ausgangs-pH-Wert zwischen 6,0 und 8,0 und einer Temperatur zwischen 25°C und 35°C aerob kultiviert, die Zellmasse abtrennt und das Enzym und aus den Zellen isoliert.

**3.** Verwendet der N-Acyl-L-prolin-Acylase gemäß Anspruch 1 zur Gewinnung von L-Prolin aus N-Acetyl-L-prolin, N-Chloracetyl-L-prolin, N-Formyl-L-prolin, N-Propionyl-L-prolin, N-Butyryl-L-prolin, N-Valeryl-L-prolin, N-Caproyl-L-prolin, N-Acetyl-D,L-prolin, N-Chloracetyl-D,L-prolin, N-Formyl-D,L-prolin, N-Propionyl-D,L-prolin, N-Butyryl-D,L-prolin, N-Valeryl-D,L-prolin oder N-Caproyl-D,L-prolin sowie L-Pipecolinsäure aus N-Acetyl-L-pipecolinsäure, N-Chloracetyl-L-pipercolinsäure, N-Acetyl-D,L-pipercolinsäure oder N-Chloracetyl-D,L-pipecolinsäure sowie L-Thiazolidin-4-carbonsäure aus N-Acetyl-L-thiazolidin-4-carbonsäure, N-Chloracetyl-L-thiazolidin-4-carbonsäure, N-Acetyl-D,L-thiazolidin-4-carbonsäure oder N-Chloracetyl-D,L-thiazolidin-4-carbonsäure sowie L-Thiazolidin-2-carbonsäure aus N-Acetyl-L-thiazolidin-2-carbonsäure, N-Chloracetyl-L-thiazolidin-2-carbonsäure, N-Acetyl-D,L-thiazolidin-2-carbonsäure oder N-Chloracetyl-D,L-thiazolidin-2-carbonsäure und zur Herstellung von N-Acetyl-L-prolin, N-Propionyl-L-prolin und N-Butyryl-L-prolin aus L-Prolin und der jeweiligen Carbonsäure.

## Claims

**1.** Microbiologically prepared
N-acyl-L-proline-acylase,
characterised by the following properties:
1) Reactivity:
it eliminates the acetyl group from N-acetyl-L-proline, wherein acetic acid and L-proline are produced as the end products, and condenses acetic acid and L-proline, wherein N-acetyl-L-proline and water are produced as end products;
2) substrate specificity:
it hydrolyses N-acetyl-L-proline,
N-chloroacetyl-L-proline,
N-formyl-L-proline,
N-propionyl-L-proline,
N-butyryl-L-proline,
N-valeryl-L-proline,
N-caproyl-L-proline,
N-acetyl-L-4-hydroxyproline,
N-chloroacetyl-L-thiazolidine-4-carboxylic acid,
N-chloroacetyl-L-thiazolidine-2-carboxylic acid,
N-chloroacetyl-L-pipecolic acid,
N-benzyloxycarbonyl-glycyl-L-proline,
glycyl-L-proline,
N-acetyl-L-alanine,
N-chloroacetyl-L-methionine and
N-chloroacetyl-L-valine;
3) optimum pH:
the optimum pH is 6.8 ± 0.5;

4) pH stability:
it demonstrates good stability at 22 °C in the pH range between pH 7.0 and pH 10.0 over a period of 3 weeks;
5) optimum temperature:
the optimum temperature is 65 °C at pH 7.5;
6) temperature resistance:
no loss in activity is detectable at 70 °C and pH7.5 after 30 minutes incubation;
7) effect of inhibitors and activators:
1,10-phenanthroline, 2-mercaptoethanol, 4-chloromercuri-benzoate, 4-hydroxymercuri-benzoate, $Hg^{2+}$, $Cu^{2+}$, $Fe^{2+}$, $Fe^{3+}$, $Sn^{2+}$, $Zn^{2+}$ and $PO_4{}^{3-}$ are inhibitors and $Co^{2+}$ and $Zn^{2+}$ are activators for the apoenzyme;
8) molecular weight:
the molecular weight is 380 000 ± 40 000 Dalton;
9) sub-units:
the molecule consists of 8 sub-units of the same size, each of 45 000 ± 5 000 Dalton;
10) $K_M$ value:
the $K_M$ value for the substrate N-acetyl-L-proline is 5 mM (30 °C, 0.1 M tris/HCl buffer, pH 7.0).

**2.** A process for obtaining N-acyl-L-proline-acylase in accordance with Claim 1,
characterised in that
Comamonas testosteroni DSM 5416 or Alkaligenis denitrificans DSM 5417 is aerobically cultivated in an aqueous nutrient medium, which contains a source of carbon and nitrogen, mineral salts and N-acetyl-L-proline as inductor, and if using Comamonas testosteroni DSM 5416 also a source of vitamins, e.g. in complex form as a yeast extract, at an initial pH between 6.0 and 8.0 and a temperature between 25 °C and 35 °C, the cell mass is separated and the enzyme is isolated from the cells.

**3.** Use of N-acyl-L-proline-acylase in accordance with Claim 1 to obtain L-proline from N-acetyl-L-proline, N-chloroacetyl-L-proline, N-formyl-L-proline, N-propionyl-L-proline, N-butyryl-L-proline, N-valeryl-L-proline, N-caproyl-L-proline, N-acetyl-D,L-proline, N-chloroacetyl-D,L-proline, N-formyl-D,L-proline, N-propionyl-D,L-proline, N-butyryl-D,L-proline, N-valeryl-D,L-proline or N-caproyl-D,L-proline and L-pipecolic acid from N-acetyl-L-pipecolic acid, N-chloroacetyl-L-pipecolic acid, N-acetyl-D,L-pipecolic acid or N-chloroacetyl-D,L-pipecolic acid and L-thiazolidine-4-carboxylic acid from N-acetyl-L-thiazolidine-4-carboxylic acid, N-chloroacetyl-L-thiazolidine-4-carboxylic acid, N-acetyl-D,L-thiazolidine-4-carboxylic acid or N-chloroacetyl-D,L-thiazolidine-4-carboxylic acid and L-thiazolidine-2-carboxylic acid from N-acetyl-L-thiazolidine-2-carboxylic acid, N-chloroacetyl-L-thiazolidine-2-carboxylic acid, N-acetyl-D,L-thiazolidine-2-carboxylic acid or N-chloroacetyl-D,L-thiazolidine-2-carboxylic acid and to prepare N-acetyl-L-proline, N-propionyl-L-proline and N-butyryl-L-proline from L-proline and the appropriate carboxylic acid.

**Revendications**

**1.** N-acyl-L-proline-acylase préparée par voie microbiologique, caractérisée par les propriétés suivantes :
1) Réactivité :
Elle élimine le groupe acétyle de la N-acétyl-L-proline, ce qui donne de l'acide acétique et de la L-proline comme produits finaux, et elle condense l'acide acétique et la L-proline, ce qui donne de la N-acétyl-L-proline et de l'eau comme produits finaux.
2) Spécificité de substrat :
Elle hydrolyse N-acétyl-L-proline N-chloracétyl-L-proline, N-Formyl-L-proline, N- propionyl-L-proline, N-butyryl-L- proline, N-valéryl-L-proline, N-caproyl-L-proline, N-acétyl-L-4-hydroxyproline, Acide N-chloracétyl-L-thiazolidine-4-carboxylique, Acide N-chloracétyl-L-thiazolidine-2-carboxylique, Acide N-chloracétyl-L-pipécolique, N-benzyloxycarbonyl-glycyl-L-proline, glycyl-L-proline, N-acétyl-L-alanine, N-chloracétyl-L-méthionine et, N-chloracétyl-L-valine.
3) pH optimal :
le pH optimal est 6,8 ± 0,5 ;
4) Stabilité au pH :
elle présente à 22 °C une bonne stabilité sur une période de 3 semaines dans un intervalle de pH compris entre pH 7,0 et pH 10,
5) Température optimale:
la température optimale est de 65 °C à un pH de 7,5,

6) Résistance à la température:
à 70 ° C et pH 7,5, après incubation de 30 minutes, on ne détecte pas de perte d'activité,
7) Influence d'inhibiteurs et d'activateurs :
Ont un effet inhibisant avant tout 1.10-phénanthroline, 2-mercaptoéthanol, 4-chloromercuribenzoate, 4-hydroxymercuribenzoate, $Hg^{2+}$ , $Cu^{2+}$ , $Fe^{2+}$ , $Fe^{3+}$ , $Sn^{2+}$ , $Zn^{2+}$ et $PO_4{}^{3-}$, ont un effet activateur sur l'apoenzyme $Co^{2+}$ et $Zn^{2+}$,
8) Poids moléculaire:
le poids moléculaire et de 380.000 ± 40.000 Dalton,
9) Sous-unités :
la molécule est constituée de 8 sous-unités de taille équivalente ayant chacune 45.000 ± 5.000 Dalton,
10) Valeur $K_M$ :
la valeur $K_m$ pour le substrat N-acétyl-L-proline est de 5 mM (30 ° C, tampon Tris-Hcl 0,1 M, pH 7,0).

**2.** Procédé d'obtention de N-acyl-L-prolineacylase selon la revendication 1, caractérisé en ce qu'on cultive de façon aérobie comamonas testostéroni DSM 5416 ou alcaligènes dénitrificans DSM 5417 dans un milieu nutritif aqueux, qui contient une source de carbone et d'azote, des sels minéraux et de la N-acétyl-L-proline comme inducteur, quand on utilise comamonas testostéroni DSM 5416 on utilise en plus une source de vitamine, par exemple sous forme complexe en tant qu'extrait de levure, à un pH de départ compris entre 6,0 et 8,0 et une température comprise entre 25 ° C et 35 ° C, on sépare la masse de cellules et on isole l'enzyme à partir des cellules.

**3.** Utilisation de la N-acyl-L-proline-acylase selon la revendication 1 pour obtenir la L-proline à partir de N-acétyl-L-proline N-chloracétyl-L-proline N-formyl-L-proline,N-propionyl-L-proline, N-butyryl-L-proline, N-valéryl-L-proline, N-caproyl-L-proline, N-acétyl-D,L-proline, N-chloracétyl-D,L-proline, N-formyl-D,L-proline, N-propionyl-D,L-proline, N-butyryl-D,L-proline, N-valéryl-D,L-proline ou N-caproyl-D,L-proline ainsi que l'acide L-pipécolique à partir de l'acide N-acétyl-L-pipécolique, acide N-chloracétyl-L-pipécolique, acide N-acétyl-D,L-pipécolique ou acide N-chloracétyl-D,L-pipécolique ainsi que l'acide L-thiazolidin-4-carboxylique à partir de l'acide N-acétyl-L-thiazolidin-4-carboxylique, acide N-chloracétyl-L-thiazolidin-4-carboxylique, acide N-acétyl-D,L-thiazolidin-4- carboxylique ou acides N-chloracétyl-D,L-thiazolidin-4-carboxylique ainsi que l'acide L-thiazolidin-2-carboxylique à partir de l'acide N-acétyl-L-thiazolidin-2-carboxylique, acide N-chloracétyl-L-thiazolidin-2-carboxylique, N-acétyl-D,L-thiazolidin-2-carboxylique ou acides N-chloracétyl-D,L-thiazolidin-2-carboxylique et pour préparer la N-acétyl-L-proline, la N-propionyl-L-proline et la N-butyryl-L-proline à partir de la L-proline et de l'acide carboxylique correspondant.